# EUROPEAN PATENT APPLICATION

(11) **EP 2 040 076 A2**
(43) Date of publication of application: **25.03.2009**
(21) Application number: 08016541.8
(22) Date of filing: 19.09.2008
(51) Int. Cl.: G01N 33/543, C07K 17/06, C07K 17/14

(54) **Substrate and method for producing the substrate**

(30) Priority: 20.09.2007 JP 2007243398
(71) Applicant: FUJIFILM Corporation, Minato-ku Tokyo (JP)
(72) Inventor: Minami, Koichi, Ashigarakami-gun Kanagawa-ken 258 (JP); Ikeda, Morihito, Ashigarakami-gun Kanagawa-ken 258 (JP); Takeuchi, Yohsuke, Ashigarakami-gun Kanagawa-ken 258 (JP)
(74) Representative: HOFFMANN EITLE

(57) **Abstract**

To enable measurements having high S/N ratios, by suppressing non specific adsorption.

A substrate that includes: a base material; and chelators which are three dimensionally covalently bound to the base material at a density of 7.8×10¹⁵/mm³ or greater and 4.5×10¹⁷/mm³ or less, or two dimensionally covalently bound to the base material at a planar density of 2.0×10¹¹/mm² or greater and 1.0×10¹³/mm² or less, is used. Metal ions are coordinately bound to the chelators, and a bioactive substance having histidine tags are coordinately bound to the metal ions. A blocking agent having ligand sets are coordinately bound to the metal ions to which the bioactive substance is not coordinately bound.

## Description

### BACKGROUND OF THE INVENTION

### Field of the Invention

The present invention is related to a substrate on which a bioactive substance is coordinately bound, and a method for producing the substrate.

### Description of the Related Art

Presently, measurements that utilize intermolecular interactions, such as immune reactions in clinical tests, are being performed. In these measurements, various bioactive substances are immobilized onto base materials, and specific binding reactions between the bioactive substances and experimental substances (target substances) are measured.

For example, analysis using protein chips is a known method for analyzing interactions between specific proteins and a known or an unknown substance. Proteins are immobilized on the surfaces of base materials in protein chips, then the protein chips are utilized. A highly effective method of immobilizing the proteins is to cause them to bind to the base materials via histidine tags (His-tags), which are added in advance during expression of the proteins. By utilizing this method, the proteins can be immobilized while the bioactivities thereof are maintained. By utilizing these chips, it is possible to screen for target substances that specifically bind to the immobilized His-tag proteins ("Global Analysis of Protein Activities Using Proteome Chips", H. Zhu et al., SCIENCE, Vol. 293, pp. 2101-2105, 2001).

Protein chips to which His-tag proteins that employ NTA-metal complexes, constituted by NTA (Nitrilotriacetic acid) and metal ions, are immobilized will be described. In these protein chips, water molecules, which are coordinately bound to two coordinate sites in each complex, are substituted by nitrogen atoms of two imidazole groups of oligohistidine residue of the His-tag proteins. Thereby, His-tag proteins are caused to specifically and unidirectionally bind to the protein chips.

In analysis of interactions among bioactive substances, such as immunological measurements, bioactive substances are caused to bind with base materials, such as plates, beads, and columns. Blocking agents are immobilized on the portions of the base materials to which the bioactive substances are not bound, so as to prevent non specific adsorption of substances, which are targets of measurement ("Antibody microarrays: An evaluation of production parameters", Proteomics, Vol. 3, pp. 254-264, 2003). If target substances are non specifically adsorbed onto base materials other than by specific binding with bioactive substances, accurate analysis cannot be performed. Therefore, it is extremely important to suppress non specific adsorption in order to perform measurements at high sensitivities, and with high S/N ratios.

It is considered that it would be possible to suppress non specific adsorption in base materials to which the aforementioned His-tag proteins are immobilized, if blocking agents are immobilized to portions thereof that the His-tag proteins are not bound to. However, the combination of His-tag proteins and NTA-metal complexes has been developed for purification by affinity columns. Therefore, the binding force thereof is not strong, and dissociation equilibrium exits. Accordingly, if immobilization of blocking agents is attempted, a new problem, that the His-tag proteins, which are immobilized via the NTA-metal complexes, gradually become separated from the base material, arises.

### SUMMARY OF THE INVENTION

The present invention has been developed in view of the foregoing circumstances. It is an object of the present invention to provide a substrate which is capable of suppressing non specific adsorption, thereby enabling measurements having high S/N ratios, and a method for producing the substrate.

A substrate of the present invention comprises:
a base material;
chelators which are three dimensionally covalently bound to the base material at a density of 7.8×10¹⁵/mm³ or greater and 4.5×10¹⁷/mm³ or less, or two dimensionally covalently bound to the base material at a planar density of 2.0×10¹¹/mm² or greater and 1.0×10¹³/mm² or less;
metal ions, which are coordinately bound to the chelators;
a bioactive substance having histidine tags, which are coordinately bound to the metal ions; and
a blocking agent having ligand sets, which are coordinately bound to the metal ions to which the bioactive substance is not coordinately bound.

It is preferable for the histidine tags to be peptides having at least six histidine residues which are close to each other.

It is preferable for the ligand sets of the blocking agent to have at least four imidazole groups; and for each molecule of the blocking agent to have one of the ligand sets.

It is preferable for the blocking agent to include one of: water soluble polymer molecules and water soluble proteins. It is preferable for the water soluble polymer molecules to be one of: polyethylene glycol, polymer molecules including phosphorylcholine groups, polysaccharides, polyvinyl alcohol, and polyhydroxy ethyl methacrylate. It is preferable for the water soluble proteins to be one of: albumin and casein.

It is preferable for the chelators to be three dimensionally covalently bound to the base material via a hydrogel; and for the hydrogel to be a carboxylic acid derivatives of dextran.

It is preferable for the chelators to be nitrilotriacetic acid derivatives.

The surface of a single substrate may have both portions to which the bioactive substance is coordinately bound and portions to which the bioactive substance is not coordinately bound.

A method for producing a substrate of the present invention comprises the steps of:
causing a bioactive substance to contact chelators, to which metal ions are coordinately bound, to coordinately bind the bioactive substance to the metal ions; and
causing a blocking agent to contact the metal ions, to coordinately bind the blocking agent to the metal ions.

It is preferable for the concentration of the blocking agent when it is caused to contact the metal ions to be within a range from 0.1nM to 20µM.

The substrate of the present invention comprises: the base material; chelators which are three dimensionally covalently bound to the base material at a density of 7.8×10¹⁵/mn³ or greater and 4.5×10¹⁷/mm³ or less, or two dimensionally covalently bound to the base material at a planar density of 2.0×10¹¹/mm² or greater and 1.0×10¹³/mm² or less; the metal ions, which are coordinately bound to the chelators; the bioactive substance having histidine tags, which are coordinately bound to the metal ions; and the blocking agent having ligand sets, which are coordinately bound to the metal ions to which the bioactive substance is not coordinately bound. Therefore, the substrate is capable of suppressing non specific adsorption of bioactive substances other than those which are coordinately bound, while suppressing separation of the bioactive substance which is coordinately bound thereto. Accordingly, measurements can be performed with high S/N ratios.

### BRIEF DESCRIPTION OF THE DRAWINGS

Figure 1 is a schematic diagram that illustrates a substrate according to a first embodiment of the present invention.
Figure 2 is a schematic diagram that illustrates a state in which a target substance is bound to the substrate of Figure 1.
Figure 3 is a schematic diagram that illustrates non specific adsorption in a case that a blocking process is not administered.
Figure 4 is a schematic diagram that illustrates non specific adsorption in a case that a blocking process is administered with a blocking agent not having ligand sets.
Figure 5 is a graph that illustrates the S/N ratios of Examples 1 through 3 and Comparative Examples 1 through 3.
Figure 6 is a graph that illustrates the S/N ratios of Example 4 and Comparative Examples 4 and 5.

### BEST MODE FOR CARRYING OUT THE INVENTION

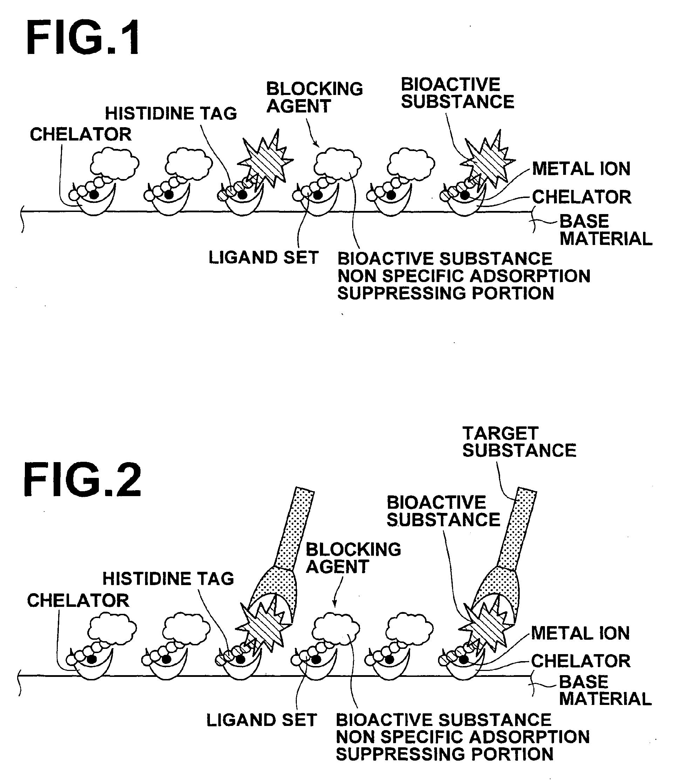

Hereinafter, the substrate of the present invention will be described with reference to the attached drawings. Figure 1 is a schematic diagram that illustrates a substrate according to a first embodiment of the present invention. The substrate of the present invention comprises: a base material; chelators which are three dimensionally covalently bound to the base material at a density of 7.8×10¹⁵/mm³ or greater and 4.5×10¹⁷/mm³ or less; metal ions, which are coordinately bound to the chelators; bioactive substance having histidine tags, which are coordinately bound to the metal ions; and a blocking agent having ligand sets, which are coordinately bound to the metal ions to which the bioactive substance is not coordinately bound. In the substrate of the present invention, the ligand portions of the blocking agent suppresses non specific adsorption onto the metal ions, and the other portions of the blocking agent suppresses non specific adsorption of the bioactive substance (hereinafter, portions of the blocking agent other than the ligand portions will be referred to as "bioactive substance non specific adsorption suppressing portions").

The chelators are three dimensionally covalently bound to the base material at a density of 7.8×10¹⁵/mm³ or greater and 4.5×10¹⁷/mm³ or less. It is preferable for the chelators to be covalently bound to the base material at a density greater than or equal to 1. 0×10¹⁶/mm³, and further preferably at a density greater than or equal to 1.0×10¹⁷/mm³. Note that it is preferable for a hydrogel to be provided on the base material, and for the chelators to be bound to the base material via the hydrogel. On the other hand, in the case that the chelators are bound to an SAM (Self Assembled Monolayer), a hydrophobic polymer, or the like, the chelators will be two dimensionally bound to the base material. In the case that the chelators are bound two dimensionally, it is preferable for the planar density thereof to be 2.0×10¹¹/mm² or greater and 1.0×10¹³/mm² or less. It is preferable for the planar density of the chelators on the base material to be greater than or equal to 5.0×10¹¹/mm², and further preferable for the planar density to be greater than or equal to 1.1×10¹²/mm².

In the case that the chelators are three dimensionally covalently bound to the base material, a chelator density of greater than 7.8×10¹⁵/mm³ will result in imidazole groups derived from the histidine tags of the bioactive substance to be coordinately bound to the metal ions, which are coordinately bound to the chelators, at a plurality of points. Therefore, the practical separation speed of the bioactive substance will dramatically decrease. Here, the "practical separation speed" refers to the speed at which the bioactive substance separates from the base material. Similarly, in the case that the chelators are two dimensionally covalently bound to the base material, a chelator density of greater than 2.0×10¹¹/mm² will promote coordinate binding at plurality of points, and the practical separation speed dramatically decreases.

The chelator density can be determined by the following methods. In the case that actual measurement is performed, the chelators are caused to covalently bind with a substrate. Thereafter, metal ions are added, and the number of metal ions which are coordinately bound to the substrate is measured by an ICP analysis apparatus or the like. The number of chelators per unit area can be determined from the number of metal ions and the area of the substrate onto which the chelators are covalently bound. In the case that calculation of the chelator density is performed, the number of chelators per unit area can be determined by utilizing a calculating software program such as CHEM 3D (by Cambridge Soft) to obtain the volume of the chelators. In the case that the volume of the chelators is obtained by a calculating software program and the chelator is NTA, for example, an estimated volume of approximately 0.3nm³ is obtained. Therefore, it is theoretically difficult to cause the chelators to be bound to the base material at a density higher than 4.5×10¹⁷/mm³ in the case that the chelators are three dimensionally bound, and at a density higher than 1.0×10¹³/mm² in the case that the chelators are two dimensionally bound.

If a target substance is caused to contact the substrate illustrated in Figure 1, the target substance specifically binds to the bioactive substance immobilized on the metal ions, as illustrated in Figure 2. Meanwhile, non specific adsorption of the target substance onto the metal ions, to which the bioactive substance is not immobilized, is suppressed by the blocking agent immobilized thereon. This suppression of non specific adsorption is a result of the bioactive substance non specific adsorption suppressing portions of the blocking agent suppressing the non specific adsorption of the bioactive substance and the ligand portions of the blocking agent suppressing non specific adsorption onto the metal ions. Accordingly, even if the blocking agent having ligand sets of the present invention did not have the bioactive non specific adsorption suppressing portions, in other words, if the blocking agent was constituted only by ligands, it is possible to suppress non specific adsorption onto the metal ions. Therefore, measurements can be performed at high S/N ratios. Of course, if the bioactive substance non specific adsorption suppressing portions are immobilized onto the blocking agent constituted only by ligands, it becomes possible to suppress both non specific adsorption onto the metal ions and non specific adsorption of the bioactive substance.

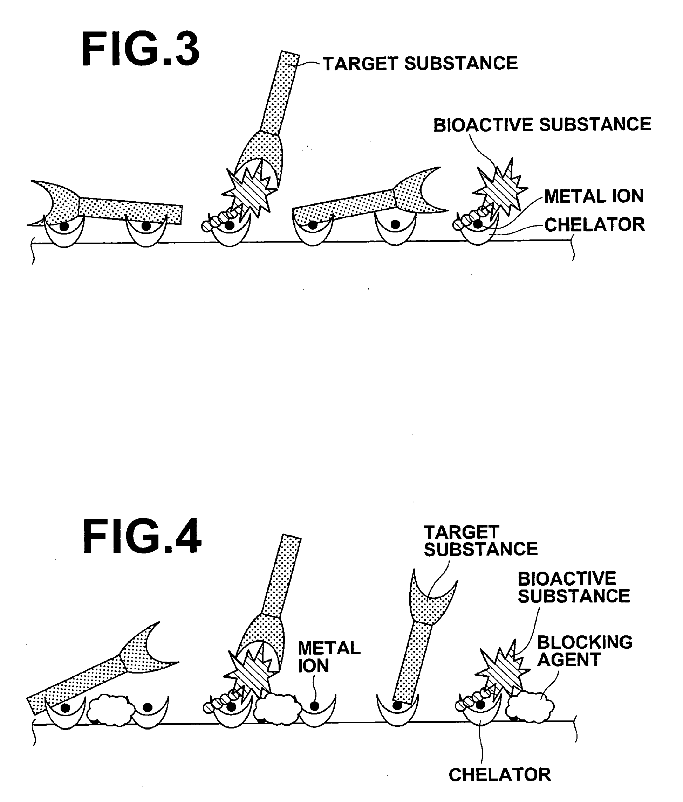

In the case that the blocking agent is not provided, the target substance binds to the bioactive substance immobilized on the metal ions, but also is non specifically adsorbed onto the metal ions which are not blocked by the ligand portions of the blocking agent and chelators, as illustrated in Figure 3. Accordingly, accurate detection of the specific binding reactions cannot be performed. Similarly, in the case that the substrate is processed with a blocking agent that does not have ligand sets, the blocking agent is not immobilized onto the metal ions, as illustrated in Figure 4. In this case, the blocking agent binds to the gaps between the chelators and the like, and does not function as a blocking agent with respect to the metal ions or the chelators. In this case as well, accurate detection of the specific binding reactions cannot be performed.

The blocking agent is immobilized to the metal ions by the ligand sets of the blocking agent. The ligand sets of the blocking agent are not particularly limited, as long as they have lone electron pairs and interact with metals. The ligands may be monodentate ligands or multidentate ligands. Specific examples of such ligands include: ethylene diamine; acetyl acetonato; glycinato; and derivatives of nitrogenous heterocyclic rings. The nitrogenous heterocyclic ring may be a monocycle or a condensed three to seven member ring including at least one nitrogen atom.

Preferably, the nitrogenous heterocyclic ring is a five member ring or a six member ring. Examples of ligands having such nitrogenous heterocyclic rings include: pyrrole; imidazole; pyrazole; oxazole; isooxazole; thiazole; isothiazole; 1,2,3-triazole; 1,2,4-triazole; 1,3,4-thiadiazole; tetrazole; pyridine; pyrazine; pyrimidine; pyridazine; 1,2,3-triazine; 1,2,4-triazine; 1,3,5-triazine; 1,2,4,5-tetrazine; azepine; azonine; quinoline; isoquinoline; acridine; phenanthridine; indole; isoindole; carbazole; benzimidazole; 1,8-naphthyridine; purine; pteridine; benzotriazole; quinoxaline; quinazoline; perimidine; cinnoline; phthaladine; 1,10-phenanthroline; phenoxazine; phenothiazine; phenazine; 8-hydroxyquinoline; 8-mercaptoquinoline; 2,2'-bipyridine; 2,2'-dipyridyl amine; di (2-picolyl amine) ; 2,2',2"-terpyridine; porphyrin; phthalocyanine; and derivatives thereof. Among the above ligands, pyrrole, imidazole, pyrazole, oxazole, thiazole, pyridine, and derivatives thereof are preferable, from the viewpoint of improving the stability of the obtained metallic complexes. Imidazole is preferable, from the viewpoint of improving the stability of the obtained metallic complexes, and from the viewpoint of suppressing exchange reactions with imidazole derived from the bioactive substance. In the case that the blocking agent is a protein, it is preferable for the ligands to be imidazole groups derived from histidine. These ligand sets may be those that have different structural types, obtained by appropriate combinations of structural types. Examples of such ligand sets include: imdazole-( )-imidazole-( )-imidazole-( ) and imidazole-imidazole-( )-imidazole-imidazole.

It is preferable for the blocking agent to have two or more and 100 or fewer ligands for each molecule thereof, in order for the blocking agent to be immobilized at a plurality of points. It is further preferable for the blocking agent to have four or more and 25 or fewer ligand sets for each molecule thereof. It is preferable for the blocking agent to have a large number of ligand sets for the purposes of promoting immobilization at a plurality of points. Appropriate lengths can be selected according to a synthesis method, the method by which the blocking agent is added, and the like.

It is preferable for the blocking agent to be water soluble polymer molecules or water soluble proteins. Albumin, casein, gelatin and the like are preferred examples of water soluble proteins. Among these, albumin and casein are preferred from the viewpoint of improving suppression of non specific adsorption. Preferred examples of water soluble polymer molecules include: polyethylene glycol; polymer molecules including phosphorylcholine groups; polysaccharides; polyvinyl alcohol; polyhydroxy ethyl methacrylate; polyacrylic acid; polyacryl amide; and polymers including geminate ions. Among these, polyethylene glycol, polymer molecules including phosphorylcholine groups, polysaccharides, polyvinyl alcohol, and polyhydroxy ethyl methacrylate are preferred from the viewpoint of improving suppression of non specific adsorption.

It is preferable for each molecule of the water soluble polymer molecules or the water soluble proteins to have one ligand. It is more preferable for each molecule of the water soluble polymer molecules or the water soluble proteins to have four or more histidines which are close to each other.

Hereinafter, each element of the substrate of the present invention and the method for producing the substrate will be described in greater detail.

### (1) Base material

The "base material" of the substrate of the present invention refers to a base material to which a bioactive substance can be immobilized. The base material may be of any desired shape, such as: a planar substrate; a substrate having protrusions and recesses; a rough substrate; particles; fine particles; rod shaped particles; a column; a mesh; and the tip of a probe. The material of the substrate may be: metals such as gold, silver, aluminum, and stainless steel; oxides such as glass, silica, alumina, titania, zirconia, and indium tin oxide; nitrides, such as silicon nitride, gallium nitride, aluminum nitride, and indium nitride; polymers such as polystyrene, polyacrylic acid ester, polyester, polycarbonate, cycloolefin polymer, and cellulose derivatives. These materials may be employed singly or in combinations.

### (2) Chelator Binding Section

A hydrogel, a self assembled monolayer, a silane coupling layer, a hydrophobic polymer film, an inorganic porous film, or an organic inorganic hybrid filmmay be used as a chelator binding layer. Alternatively, combinations of the above may be used, in which a self assembled monolayer or a silane coupling layer is formed on the surface of the base material, then a hydrogel or a hydrophobic polymer film is formed on the self assembled monolayer or the silane coupling layer. The mobility of the chelators is high in an aqueous environment if they are bound via a hydrogel. Therefore, binding the chelators to the base material via a hydrogel is preferable from the viewpoint of promoting binding of the bioactive substance and the blocking agent at a plurality of points. Hereinafter, a two dimensional covalent binding state and a three dimensional covalent binding state of the chelators will be described in detail.

### (2-1) Two Dimensional Binding Section

Various methods for binding the chelators onto the base material two dimensionally may be used. Examples of such methods include: that in which the chelators are bound via a self assembled monolayer or a silane coupling layer which is formed on the surface of the base material; that in which the chelators are bound via a hydrophobic polymer film which is coated onto the surface of the base material; that in which the chelators are bound via a Langmuir Blodget film which is transferred onto the surface of the base material; and that in which the chelators are bound via a processed surface layer, the surface processed layer being formed by oxidizing, reducing, or hydrolytically degrading the vicinity of the surface of the base material.

As a specific example, a self assembled monolayer with respect to metal surfaces can be favorably utilized. Coating methods for metal films using self assembled monolayers have been developed by Professor Whitesides of Harvard University et al. The details thereof are reported in Chemical Review, Vol. 105, pp. 1103-1169, 2005, for example. In the case that gold is used as the metal, an alkanethiol derivative represented by General Formula A-1 may be employed as a self assembled monolayer forming compound. In General Formula A-1, n represents an integer within a range from 3 to 20, and more preferably within a range from 6 to 15, and X represents a functional group. Thereby, a monomolecular film having uniform orientation is formed in a self assembling manner, based on As-S bonds and van der Waal forces among the alkyl chains.

X- (CH₂)ₙ-SH A-1

Specific examples of the self assembled monolayer forming compound include: 6-amino-1-hexanethiol; 11-hydroxy-1-undecanethiol; 10-carboxy-1-decanethiol; 7-carboxy-1-heptanethiol; 16-carboxy-1-mercaptohexadecanethiol; and 11-hydroxy-1-undecanethiol. The self assembled monolayer can be produced by a simple technique of immersing a gold substrate within an alkanethiol derivative solution. It is possible to coat a metal surface with an organic layer having amino groups, by forming a self assembled monolayer using a compound represented by General Formula A-1, in which X is NH₂.

As another example, with respect to base materials having thin films of metal oxides such as silica and nitrides such as silicon nitride thereon, the chelators can be bound thereto via a silane coupling layer, which is formed by a silane coupling agent. A compound represented by General Formula A-2 may be employed as the silane coupling agent. In General Formula A-2, X represents a functional group; L represents a linker portion including a carbon chain, such as straight chain, a branched chain, and a cyclic chain; R represents a hydrogen atom or an alkyl group having a carbon number from 1 to 6; and Y represents a hydrolysis group. In addition, m and n each represents an integer from 0 to 3, and m+n=3. By using the silane coupling agent represented by General Formula A-2, covalent bonds of metal (silicon) - oxygen - silicon - carbon can be formed, and the surface of the base material can be coated with functional groups.

X-L-Si-(Rₘ)Yₙ A-2

Here, examples of the hydrolysis group Y include: alkoxy groups; halogen; and acyloxy groups. Specific examples include: methoxy groups; ethoxy groups; and chlorine. Specific examples of the silane coupling agent include: γ-aminopropyl trimethoxysilane; N-β (aminoethyl) γ-aminopropyl trimethoxysilane; γ-aminopropyl methyl diethoxy silane; y-mercaptopropyl trimethoxy silane; and γ-glycidoxypropyl triethoxy silane. Known reaction methods may be used for the silane coupling agent, for example, those disclosed in Silane Coupling Agents: Effects and Uses, Science and Technology Co.

The functional groups (X) of the self assembled monolayer forming compound and the silane coupling agent is not particularly limited as long as it bindss with the bioactive substance. Examples of such functional groups include: amino groups; carboxyl groups; hydroxyl groups; aldehyde groups; thiol groups; isocyanate groups; isothiocyanate groups; epoxy groups; cyano groups; hydrazino groups; hydrazide groups; vinyl sulfone groups; vinyl groups; and derivatives thereof. These functional groups may be used singly or in combination.

### (2-2) Three Dimensional Binding Section

It is preferable for a hydrogel to be employed as a three dimensional binding section. Examples of hydrogels include: gelatin; agarose; chitosan; dextran; carrageenin; alginic acid; starch; cellulose; and derivatives thereof, such as carboxymethyl derivatives. Examples of hydrogels also include: water swelling organic polymers, such as polyvinyl alcohol, polyacrylic acid, polyacryl amide, polyethylene glycol, and derivatives thereof.

Further, it is possible to use carboxymethyl group containing synthetic polymers and carboxymethyl group containing polysaccharides as the hydrogel in the present invention. Examples of carboxymethyl group containing synthetic polymers include: polyacrylic acid; polymethacrylic acid; and copolymers of polyacrylic acid and polymethacrylic acid. Examples of such copolymers include: methacrylic acid copolymers as disclosed in Japanese Unexamined Patent Publication Nos. 59(1984)-053836 (page 3, line 20 - page 6, line 49) and 59(1984)-071048 (page 3, line 41 - page 7, line 54); acrylic acid copolymers; itaconic acid copolymers; crotonic acid copolymers; maleic acid copolymers; partially esterified maleic acid copolymers; and polymers having hydroxyl groups, to which anhydrides are added. Examples of carboxylmethyl group containing polysaccharides include: natural plant extracts; products of microbial fermentation; synthetics formed by enzymes; and chemically synthesized compositions. Specific examples include: hyaluronic acid; chondroitin sulfate; heparin; dermatan sulfate; carboxymethyl cellulose; carboxyethyl cellulose; cellouronic acid; carboxymethyl chitin; carboxymethyl dextran; and carboxymethyl starch. Commercially available compounds may be used as the carboxymethyl group containing polysaccharides. Examples of such commercially available compounds include: CMD, CMD-L and CMD-D40 (manufactured by Meito Sangyo Co., Ltd.), which are carboxymethyl dextrans; carboxymethylcellulose sodium (manufactured by Wako Pure Chemical Industries, Ltd.), and sodium arginine (manufactured by Wako Pure Chemical Industries, Ltd.).

The molecular weight of the hydrogel to be used in the present invention is not particularly limited. It is preferable for the molecular weight of the hydrogel to be within a range from 200 to 5,000,000. It is more preferable for the molecular weight of the hydrogel to be within a range from 100,000 to 2,000,000.

The hydrogel may be directly bound or indirectly bound to the base material. Alternatively, the hydrogel may be directly formed on the base material from water containing monomers. Further, the hydrogel may be cross linked. Cross linking of hydrogels is obvious to those skilled in the art.

It is preferable for the film thickness of the hydrogel, to be bound to a sensor surface of a biosensor to be described later, to be within a range from 1nm to 0.5mm in solution. It is more preferable for the film thickness of the hydrogel to be within a range from 1nm to 1µm. If the film thickness is too thin, the amount of bioactive substance which is immobilized on the hydrogel decreases, and interactions with the target substance become less likely to occur. On the other hand, if the film thickness is too thick, the target substance becomes diffused within the film, which is not preferable. The film thickness of the hydrogel in solution can be evaluated by an AFM, ellipsometry, or the like.

### (2-3) Immobilization of the Hydrogel onto the Base Material

The hydrogel may be immobilized onto the base material either directly or via a reaction layer, such as the self assembled monolayer and the silane coupling layer, which were described in the section regarding the two dimensional binding section. In the case that a polymers that contain carboxyl groups are utilized as the hydrogel, the hydrogel can be immobilized onto the base material by activating the carboxyl group. The hydrogel can be immobilized on the base material, by causing the polymers containing the carboxyl groups, which have been activated by these methods, to react with functional groups of the base material, such as amino groups, or functional groups of the self assembled monolayer or the silane coupling layer. Known methods can be used to activate the polymers containing carboxyl groups. Examples of such methods include: a method in which carboxyl groups are activated using carbodiimides such as 1-(3-Dimethylaminopropyl)-3 ethylcarbodiimide (EDC), N,N'-dicyclohexylcarbodiimide (DCC), and N,N'diisopropylcarbodiimide (DIC), and active ester generating compounds, such as N-Hydroxysuccinimide (NHS), 3-hydroxy-3, 4-dihydro-4-oxo-1, 2, 3-benzotriazine (HODhbt), pentafluorophenol, and p-nitrophenol; and methods in which carboxyl groups are activated using the carbodiimides or the active ester generating compounds alone. Further, a method in which EDC and NHS are used to activate the carboxyl groups, and a method in which EDC alone is used to activate the carboxyl groups, may also be favorably used. The hydrogel can be immobilized on the base material, by causing the polymers containing the carboxyl groups, which have been activated by these methods, to react with the base material, which has amino groups.

The polymer having the carboxyl groups which have been actively esterified can be caused to react with the base material as a solution. Alternatively, the polymer may be cause to react with the base material in a state in which a thin film is formed on the base material by the spin coat method or the like. The reaction with the base material having a thin film thereon is preferred.

As described above, it is preferable for the polymer having the carboxyl groups which have been actively esterified to react with the base material in a thin film state. Known methods may be used to form the thin film on the base material. Specific examples of such methods include: the extrusion coating method; the curtain coating method; the casting method; the screen printing method; the spin coat method; the spray coat method; the slid bead coating method; the slit and spin method; the slit coating method; the dye coating method; the dip coat method; the knife coat method; the blade coating method; the flow coating method; the roll coat method; the wire bar coating method; and the transfer printing method. These thin film forming methods are described in: Y. Harasaki, "Advances in Coating Techniques", Total Technology Center, 1988; "Coating Techniques", Technological Information Society, 1999; "Water Based Coating Techniques", CMC, 2001; "Film Forming of Advanced Organic Thin Films", S. B. Research Co., Ltd., 2004; A. Iwamori, "Polymer Surface Processing", Gihodo Publisihing, 2005; and the like. In the present invention, the spray coat method or the spin coat method is preferred as the method for forming the thin film on the base material, and the spin coat method is particularly preferred.

### (3) Chelators

Various chelate solutions may be used as compounds to become the chelators. Preferred examples include polydentate chelators, such as: nitrilotriacetic acid (NTA); iminodiacetic acid (IDA); phenanthroline; terpyridine; bipyridine; triethylene tetraamine; bi (ethylene triamine); tris (carboxy methyl) ethylene diamine; diethylene triamine pentaacetic acid; polypyrazolyl boric acid; 1,4,7-triazacyclononane; dimethyl glyoxime; diphenyl glyoxime; and derivatives thereof. Among these, NTA, iminodiacetic acid, and derivatives thereof are particularly preferred. In the case that the hydrogel having carboxyl groups is provided on the surface of the base material, the chelators can be bound to the hydrogel, by activating the carboxyl groups, then causing a compound which is to become the chelator to react therewith, for example.

It is preferable for an organic solvent to be employed during binding of the chelators. Use of the organic solvent enables three dimensional binding of the chelators at a density of 7.8×10¹⁵/mm³ or greater and 4.5×10¹⁷/mm³ or less, or two dimensional binding at a planar density of 2.0×10¹¹/mm² or greater and 1.0×10¹³/mm² or less.

Examples of organic solvents include: dimethylsulfoxide; N,N-dimethylformamide;; N,N-dimethylacetoamide; acetonitrile; N-methylpyrrolidone; acetone; methyl ethyl ketone; methanol; ethanol; isopropyl alcohol; sec-butyl alcohol; tert-butyl alcohol; butyl cellosolve; tetrahydrofuran; and diglyme. From the viewpoints of solubility of chelators and suppression of side reactions, dimethylsulphoxide and N,N-dimethyl formamide are preferred.

### (4) Metal Ions

Any metal ion may be employed as the metal ions, as long as they form unsaturated metallic complexes. From the viewpoint of stability of obtained metallic complexes, transitional metal ions are preferred. Specifically, appropriate metal ions may be selected from among a group consisting of: Ni(II); Cu(I); Cu(II); Co(II); Co(III); Fe(II); Fe(III); and Ga(III), according to the type of chelator. Among these, Ni(II), Cu(II), Co(III), and Fe(III) are preferred as the metal ions, and Ni(II) and Cu(II) are particularly preferred. Note that the binding force of metal ions varies according to valence. In the case of Co (II) and Fe(II), the oxidation number of metal ions can be changed by the oxidation reduction method described in paragraphs [0037] and [0038] of Japanese Unexamined Patent Publication No. 6(1994)-157600, to vary the binding forces thereof.

### (5) Bioactive Substance

Examples of the bioactive substance include: immunoproteins; non immune proteins; immunoprotein binding proteins; glycobinding proteins; enzymes; nucleic acids; low molecular weight organic compounds; sugar chains that recognize sugars; fatty acids; fatty acid esters; microorganisms; and viruses. The histidine tags of the bioactive substance are preferably peptides having four or more histidine residues, which are amino acids having imidazole groups. The histidine tags may be added to the bioactive substance by chemical bonds, or by genetic engineering, in the case that the bioactive substance is a protein. If there are many imidazole groups in proximity to each other, the bioactive substance can be immobilized on greater numbers of chelators and metal ions at a plurality of points. Therefore, it is more preferable for the histidines to be peptides having six or more histidine residues. It is further preferable for the histidines to be peptides having ten or more histidine residues, from the viewpoint of increasing the strength of immobilization. However, if the number of histidines becomes too great, the activity of the bioactive substance becomes likely to be inhibited. Therefore, the number of histidines is preferably 100 or less. Here, the histidine residues which are in proximity to each other refer to a state in which zero to three amino acids are present among the histidines.

### (6) Immobilization of the Bioactive Substance

The bioactive substance is immobilized, by causing a solution that includes the bioactive substance to contact the base material. In the case that the bioactive substance includes the imidazole groups derived from the hisitidine tags, coordinate bonds are formed with the metal ions to form complexes, thereby immobilizing the bioactive substance.

For example, in the case that NTA is utilized as the chelators, and metal ions capable of six coordinate bonds are added, (1) three carboxyl groups and one nitrogen atom of the NTA occupy four of the six coordinate sites, (2) a histidine tag occupies one of the two remaining coordinate sites, and (3) a water molecule or a hydroxide ion occupies the last remaining coordinate site, to form six coordinate complexes.

In the case that iminodiacetic acid is utilized as the chelators, and metal ions capable of six coordinate bonds are added, (1) two carboxyl groups and one nitrogen atom of the iminodiacetic acid occupy three of the six coordinate sites, and (2) histidine tags of the bioactive substance and (3) water molecules or hydroxide ions occupy the remaining three coordinate sites, to form six coordinate complexes.

Here, metal ions capable of six coordinate bonds have been described as examples. However, the number of coordinate sites may be seven or greater, or five or less. In addition, the carboxyl groups that form the complexes need not be provided by a single chelator, but from a plurality of chelators.

Further, it is preferable to form the substrate such that the surface of a single substrate has both portions to which the bioactive substance is coordinately bound and portions to which the bioactive substance is not coordinately bound. Here, "portions to which the bioactive substance is not coordinately bound" does not refer to gaps at which the bioactive substance was not immobilized even though the immobilization process was administered, but rather portions (reference portions) to which only a blocking process was administered. An example of a substrate having portions to which the bioactive substance is coordinately bound and portions to which the bioactive substance is not coordinately bound is that in which a great number of measurement regions, in which bioactive substances are immobilized, are discretely provided on the surface of the substrate, and the blocking process is administered to the other regions thereof. Because the blocking agent is immobilized onto the portions to which the bioactive substance is not immobilized, both signals due to specific binding and those due to non specific adsorption can be suppressed. Accordingly, a background signal value for the substrate itself can be obtained.

### (7) Immobilization of the Blocking Agent

The blocking agent is immobilized in the same manner as the bioactive substance. That is, a solution containing the blocking agent is caused to contact the substrate, and the blocking agent is immobilized thereon by the ligand sets of the blocking agent coordinately binding with the metal ions to form complexes. When the blocking agent having the ligand sets is added, (1) the chelators, (2) the ligand sets of the blocking agent, and (3) in some cases, water molecules or hydroxide ions, coordinately bind with the metal ions, to form the complexes.

Note that it is preferable for the blocking agent to be immobilized to portions of the substrate to which the bioactive substance is immobilized, and to portions of the substrate to which the bioactive substance is not immobilized. Here, the "portions of the substrate to which the bioactive substance is not immobilized" does not refer to gaps at which the bioactive substance was not immobilized even though the immobilization process was administered, but refers to portions to which only a blocking process was administered, as described above.

It is preferable for the concentration of the blocking agent to be within a range from 0.1nM to 20µM. This concentration is weaker than that of blocking agents which are generally used to prevent non specific adsorption in immunological measurement methods. However, even if the concentration is this weak, because the blocking agent has ligand sets that immobilize the blocking agents to the metal ions, effective blocking becomes possible. In addition, if the concentration of the blocking agent is higher than 20µm, as in general immunological measurement methods, the ligand sets of the blocking agents and imidazole ligands of the bioactive substance will become immobilized to each other due to exchange reactions. This will accelerate the separation speed of the bioactive substance, which is not favorable. However, if this effect is not considered to be detrimental, the blocking agent may be of higher concentrations.

### (8) Production of the Substrate

The substrate of the present invention may be produced by the following steps. In the case that a glass plate is used as the base material, for example, first, amino groups are introduced to the surface of the glass plate, by causing γ-aminopropyl triethoxysilane to react with the glass plate. Then, a hydrogel is formed on the silane coupling layer. The surface of the solid phase substrate is coated by the amino groups of the silane coupling agent. Therefore, in the case that the hdrogel contains carboxyl groups, the hydrogel can be immobilized onto the base material by activating the carboxyl groups.

Next, the chelators are caused to bind to the hydrogel. The binding of the chelators in the case that the hydrogel has carboxyl groups, for example, can be performed by activating the carboxyl groups, then causing a compound which is to become the chelators to react therewith within an organic solvent.

Thereafter, the metal ions are coordinately bound to the chelators. Next, the bioactive substance is caused to contact the metal ions to become immobilized thereon. Finally, the blocking agent having the ligand sets is caused to contact the metal ions, to cause the blocking agent to become immobilized onto the metal ions on which the bioactive substance is not immobilized.

### (9) Application of the Substrate of the Present Invention

The substrate of the present invention may be employed in biosensors. Here, the term "biosensor" is interpreted to have a broad meaning, and refers to an sensor that converts interactions among biological molecules into signals, such as electric signals, to measure and/or detect target substances.

Generally, biosensors are constituted by receptor sites that recognize chemical substances as detection targets, and transducer sites that convert physical or chemical changes at the receptor sites into signals such as electric signals. Substances that have affinities for each other within living organisms include: enzymes and substrates; enzymes and coenzymes; antigens and antibodies; and hormones and receptors. In biosensors, one of a pair of substances that have affinities for each other is immobilized onto a base material and utilized as receptor sites, to selectively measure the other of the pair of substances.

The substrate of the present invention may also be applied to protein chips or microarrays having a great number of discrete measurement regions, at which bioactive substances are immobilized, on the surface of a substrate. Further, the substrate of the present invention may be applied to biosensors that utilize substrates which are not flat, such as fine particles and base materials having protrusions and recesses.

Hereinafter, examples of the substrate of the present invention will be described.

### (Example 1)

### (Production of Aminated Glass Slide)

A solution containing γ-aminopropyl triethoxysilane (by Tokyo Kasei Industries, KK) at 0.1 weight % in toluene was prepared. UV cleansing was administered on a glass slide (by Matsunami Glass Industries, Ltd.), then the glass slide was immersed in the γ-aminopropyl triethoxysilane solution for 12 minutes at 60°C. The glass slide was removed from the solution, then cleansed with toluene, ethanol, and ultrapure water in this order, then underwent a baking process for one hour at 120°C.

### (Formation of CMD Covered Slide)

CMD (by Meito Sangyo Co. Ltd., molecular weight: 1,000,000) was dissolved in ultrapure water such that the concentration thereof was 0.1 weight %. Then, 100µL of a 40mM EDC (1-(3-dimethylaminopropyl)-3-ethylcarbodiimide) and 10mM NHS (N-hydroxysuccinimide) solution was added to the CMD solution. The activated esterified CMD solution was dripped onto the aminated glass slide, and spin coating was performed for 45 seconds at 7000rpm. After the spin coat process, the glass slide was laid still for one hour at room temperature, then washed by a 0.1M NaOH solution and ultrapure water, in this order.

### (Production of Three Dimensionally Bound NTA Slide)

300µL of a solution, in which 1mmol of EDC and 0.2mmol of NHS was added to 1mL of DMSO was placed on the CMD coated slide, and caused to react therewith for 30 minutes within a DMSO atmosphere. The solution was removed, and the solide was washed with DMSO once. Thereafter, the slide was caused to react with a solution, which was obtained by adding 0.1mmol of AB-NTA (by Dojindo Chemicals) to 1ml of DMSO, for 12 hours. Thereafter, the solution was removed, and the slide was washed once with ultrapure water, to obtain the three dimensionally bound NTA slide.

### (Production of Blocking Agent)

200µL of a solution containing 4mM of a C terminal carbamoylized His6 peptide (2HN-(His)6-CONH2 by NeoMPS) in PBS, 200µL of a solution containing 400nM of BSA (by Sigma Aldrich) in PBS, 200µL of an aqueous 0.8M EDC solution, and 200µL of an aqueous 0.2M NHS solution were mixed, and caused to react overnight at room temperature. The reaction solution was purified with PD-10 (by GE Healthcare Bioscience) to remove free His6 peptides, to produce BSA to which the His6 binding process has been administered (hereinafter, referred to as His-BSA). The concentration of BSA after purification was measured using the BCA method, and a 100nM solution in PBS was prepared based on the measured value.

### (Binding of His tag Proteins and Blocking Process)

An aqueous 1mM CuCl₂ solution was placed on the three dimensionally bound NTA slide, and laid still for three minutes. Thereafter, the solution was removed, and the slide was washed twice with ultrapure water. Next, 5µL of an E. coli lysate containing 20nM of histidine tagged GFP (by Upstate Co., hereinafter, referred to as His GFP) was spotted at a plurality of locations on the slide. The slide was laid still in a moist chamber for 30 minutes at room temperature. The spotted solution was removed, and the slide was washed twice with PBS. Then, a 100nM His BSA PBS solution was placed on the entire surface of the three dimensionally bound NTA slide, and laid still in a moist chamber for 30 minutes at room temperature. Thereafter, the His BSA solution was removed, and the slide was washed twice with PBS.

### (Analysis of Binding Amount of Labeled Compound and Non Specific Adsorption)

A PBS solution containing anti GFP antibodies (by Rockland Immunochemicals) labeled with Cy5 (by GE Healthcare Bioscience) at a concentration of 0.1mg/mL was prepared. The GFP antibody solution was placed on the entire surface of the NTA slide, to which the blocking process was administered, and laid still within a moist chamber for 30 minutes at room temperature. The antibody solution was removed, the slide was washed three times with PBS, then dried by nitrogen gas. The slide, to which the anti GFP antibodies were placed in contact, was observed using FLA-8000 (by FUJIFILM Corp.), and the fluorescence intensities emitted at a wavelength of 675nm, induced at the spotted portions by excitation light beams having wavelengths of 635nm, was measured. The amount of binding of the GFP antibodies and the amount of non specific adsorption were estimated from the detected intensities. A signal to noise ratio was calculated with an average signal intensity of the peripheries of the portions that were spotted with the lysate containing His GFP as noise (the amount of non specifically adsorbed antibodies: N) and the average signal intensity of the spotted portions as signal (amount of bound antibodies: S).

### (Example 2)

Preparation of the substrate and analysis of the S/N ratio were performed in the same manner as for Example 1, except that casein (by Sigma Aldrich) was employed instead of BSA during production of the blocking agent.

### (Example 3)

Preparation of the substrate and analysis of the S/N ratio were performed in the same manner as for Example 1, except that the concentration of His BSA used in the blocking process was set to 300µM.

### (Comparative Example 1)

Preparation of the substrate and analysis of the S/N ratio were performed in the same manner as for Example 1, except that the blocking process was not performed.

### (Comparative Example 2)

Preparation of the substrate and analysis of the S/N ratio were performed in the same manner as for Example 1, except that unprocessed BSA, to which His6 peptides were not bound, was used in the blocking process instead of the His BSA.

### (Comparative Example 3)

Preparation of the substrate and analysis of the S/N ratio were performed in the same manner as for Example 1, except that ultrapure water was used as the reaction solvent during the AB-NTA process instead of DMSO.

### (Example 4)

### (Production of Two Dimensionally Bound NTA Slide)

A 3nm chrome film and a 20nm gold film were formed on a glass plate (50mm × 50mm) by sputtering. 9.2mg of 11-hydroxy-1-undecanethiol (by Aldrich), 1.4mg of 16-mercaptohexadecane (by Aldrich), 2mL of ultrapure water, and 8mL of ethanol were mixed at 40°C. The surface of the gold sputtered glass plate was washed by an UV/ozone process. The glass plate was immersed in the above thiol solution, and caused to react for one hour at 40°C. Thereafter, the glass plate was washed once each with ethanol and ultrapure water. Next, the glass plate as caused to react with AB-NTA in DMSO, to produce the two dimensionally bound NTA slide.

### (Binding Assay)

The glass plate was caused to contact a CuCl₂ solution, a lysate containing the histidine tagged GFP, an His BSA solution, and Cy5 labeled anti GFP antibodies as in the first example.

### (Analysis of Binding Amount of Labeled Compound and Non Specific Adsorption)

90µL of a 10mM glycine buffer (pH 1.5) was caused to contact the portions of the glass plate where the lysate containing His GFP was spotted to recover the bound Cy5 labeled antibodies, which were then mixed in 10µL of a 1M sodium bicarbonate buffer. The amount of bound Cy5 labeled antibodies was quantified from the fluorescent intensity of the recovered solution (binding amount of antibodies: S). The amount of Cy5 labeled antibodies which was recovered from the portions of the slide that were not spotted with the lysate containing His GFP (non specific adsorbed amount: N) was quantified, and the S/N ratio was calculated.

### (Comparative Example 4)

Preparation of the substrate and analysis of the S/N ratio were performed in the same manner as for Example 4, except that unprocessed BSA, to which His6 peptides were not bound, was used in the blocking process instead of the His BSA.

### (Comparative Example 5)

Preparation of the substrate and analysis of the S/N ratio were performed in the same manner as for Example 4, except that ultrapure water was used as the reaction solvent during the AB-NTA process instead of DMSO.

### (Example 5)

Preparation of the substrate was performed in the same manner as for Example 1, except that:
1) CMD (by Meito Sangyo Co. Ltd., molecular weight: 1, 000, 000) was dissolved in ultrapure water such that the concentration thereof was 1 weight %. Then, 1mL of a 10mM EDC (1-(3-dimethylaminopropyl)-3-ethylcarbodiimide) was added to the CMD solution. The activated esterified CMD solution was dripped onto the aminated glass slide, and spin coating was performed for 45 seconds at 7000rpm. After the spin coat process, the glass slide was laid still for one hour at room temperature, then washed by a 0.1M NaOH solution and ultrapure water, in this order.
2) 300µL of a solution, in which 1mmol of EDC and 0.2mmol of NHS was added to 0. 94mL of DMSO and 0.06mL of DBU (by Tokyo Kasei), was placed on the CMD coated slide, and caused to react therewith for 30 minutes within a DMSO atmosphere. The solution was removed, and the slide was washed with DMSO once. Thereafter, the slide was caused to react with a solution, which was obtained by adding 0.1mmol of AB-NTA (by Dojindo Chemicals) to 1ml of DMSO, for 2 hours. Thereafter, the solution was removed, and the slide was washed several times with ultrapure water, to obtain the three dimensionally bound NTA slide.
3) In the process for binding His tag proteins and the blocking process, 20µM of an HHHHHH peptide (H represents histidine, and HHHHHH indicates that six histidines are linked together) by Operon was used as the blocking agent instead of His BSA.

### (Example 6)

The substrate was prepared in the same manner as for Example 1, except that in the process for binding His tag proteins and the blocking process, 20µM of an HHHHHHHH peptide (H represents histidine, and HHHHHHHH indicates that eight histidines are linked together) by Operon was used as the blocking agent instead of His BSA.

### (Example 7)

The substrate was prepared in the same manner as for Example 1, except that in the process for binding His tag proteins and the blocking process, 20µM of an HHHHHHHHHH peptide (H represents histidine, and HHHHHHHHHH indicates that ten histidines are linked together) by Operon was used as the blocking agent instead of His BSA.

### (Example 8)

The substrate was prepared in the same manner as for Example 1, except that in the process for binding His tag proteins and the blocking process, 20µM of an HSHSHSHSHSHSHSHSHSHS peptide (H represents histidine, S represents serine, and HSHSHSHSHSHSHSHSHSHS indicates that ten HS's, which are H and S bound to each other, are linked together) by Operon was used as the blocking agent instead of His BSA.

### (Example 9)

The substrate was prepared in the same manner as for Example 1, except that in the process for binding His tag proteins and the blocking process, 20µM of an HKHKHKHKHKHK peptide (H represents histidine, K represents lysine, and HKHKHKHKHKHK indicates that six HK's, which are H and K bound to each other, are linked together) by Operon was used as the blocking agent instead of His BSA.

### (Example 10)

The substrate was prepared in the same manner as for Example 1, except that in the process for binding His tag proteins and the blocking process, 20µM of an HCHCHCHCHCHC peptide (H represents histidine, C represents cysteine, and HCHCHCHCHCHC indicates that six HC's, which are H and C bound to each other, are linked together) by Operon was used as the blocking agent instead of His BSA.

### (Measurement of Chelator Density)

After the AB-NTA is bound to the media, an aqueous 0.1M NiCl₂ solution was added. The solution was removed after 10 minutes, then the media were washed twice with ultrapure water. Two extractions were performed with 5ml of a 50mmol/L EDTA solution. The extracted liquids were combined and measured with an ICP analyzing apparatus to quantify the amounts of Ni. The amounts of Ni were converted to amounts of NTA. In the case of the three dimensionally bound NTA slides, the amounts of Ni were divided by the volume of CMD (height measured by AFM) to obtain NTA densities. In the case of the two dimensionally bound NTA slides, the amounts of Ni were divided by the areas thereof, to obtain NTA densities.

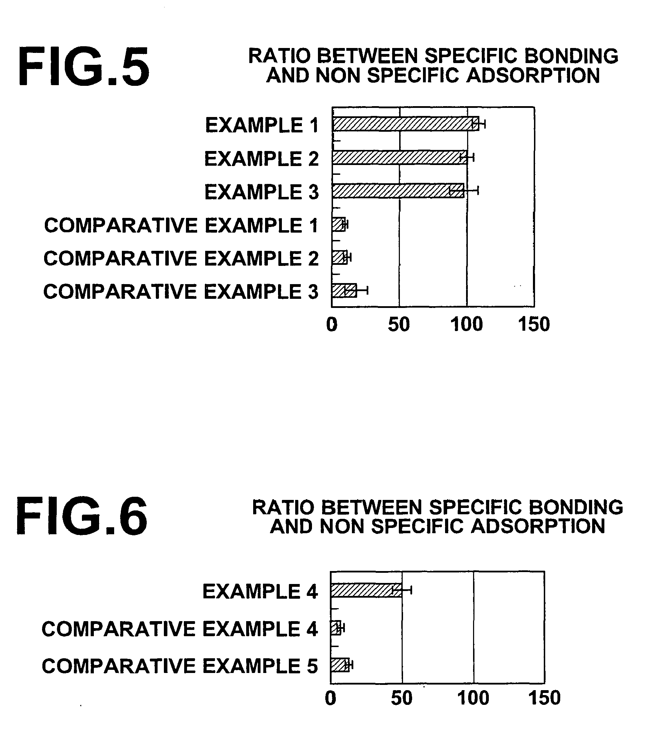

The measurement results of chelator densities are illustrated in Tables 1 and 2 below. The ratio between specific binding and non specific adsorption (S/N ratio) are illustrated in Figures5 and 6. Note that S/N ratios, which are averages of S/N ratios obtained at four points, and standard deviations are illustrated in Figures 5 and 6. In addition, the measurement results of NTA density and S/N ratios for Examples 5 through 10 are illustrated in Table 3. Note that the S/N ratios of Table 3 are averages of S/N ratios obtained at four points.

**TABLE 1**

| | Blocking Agent | NTA Density (per mm³) |
|---|---|---|
| Example 1 | His BSA | 3.5×10¹⁶ |
| Example 2 | His Casein | 3.7×10¹⁶ |
| Example 3 | 300µM His BSA | 3.9×10¹⁶ |
| Comparative Example 1 | N/A | 3.7×10¹⁶ |
| Comparative Example 2 | Unprocessed BSA | 4.0×10¹⁶ |
| Comparative Example 3 | His BSA | 6.5×10¹⁶ |

**TABLE 2**

| | Blocking Agent | NTA Density (per mm²) |
|---|---|---|
| Example 4 | His BSA | 4.3×10¹¹ |
| Comparative Example 4 | Unprocessed BSA | 4.6×10¹¹ |
| Comparative Example 5 | His BSA | 3.2×10¹¹ |

**TABLE 3**

| | Blocking Agent | NTA Density (per mm³) | Specific Binding/Non Specific Adsorption Ratio |
|---|---|---|---|
| Example 5 | HHHHHH | 4.1×10⁶ | 103 |
| Example 6 | HHHHHHHH | 4.3×10¹⁶ | 108 |
| Example 7 | HHHHHHHHHH | 3.7×10¹⁶ | 129 |
| Example 8 | HSHSHSHSHSHSHSHSHSHS | 4.5×10¹⁶ | 131 |
| Example 9 | HKHKHKHKHKHK | 4.0×10¹⁶ | 119 |
| Example 10 | HCHCHCHCHCHC | 4.2×10¹⁶ | 87 |

As is clear from Tables 1 and 2 and Figures 5 and 6, Examples 1 through 4, which were processed with the blocking agents having the His tags (ligand sets), had higher S/N ratios than Comparative Example 1, which did not undergo a blocking process, and Comparative Examples 2 and 4, which underwent blocking processes with blocking agents that did not have His tags. In the case of three dimensionally bound NTA slides, the S/N ratio of the Examples were approximately 10 times greater than those of the Comparative Examples. In the case of the two dimensionally bound NTA slides, the S/N ratio of the Example was approximately 8 times greater than those of the Comparative Examples. Comparative Examples 3 and 5 were cases in which NTA densities are low. In these cases as well, the S/N ratios were approximately two times greater than those of Comparative Examples 1, 2, and 4. However, when compared against the S/N ratios of Example 1 and Example 4, the S/N ratios of Comparative Examples 3 and 5 were less than a sixth and less than a quarter thereof, respectively.

Also, as is clear from Table 3, even in cases that the blocking agent is constituted only by ligand sets, non specific adsorption onto the metal ions are suppressed, and therefore measurements at high S/N ratios are possible. From Examples 5 through 7, it can be seen that peptides having continuous histidine groups also exhibit blocking effects. From Examples 8 through 10, it can be seen that blocking effects are exhibited in cases that the histidine residues are not continuous, but are close to each other. It can also be seen that cysteine and lysine, which interact with the metal ions, can also be used in combination with histidines.

## Claims

1. A substrate, comprising:
a base material;
chelators which are three dimensionally covalently bound to the base material at a density of 7.8×10¹⁵/mm³ or greater and 4.5×10¹⁷/mm³ or less, or two dimensionally covalently bound to the base material at a planar density of 2.0×10¹¹/mm² or greater and 1.0×10¹³/mm² or less;
metal ions, which are coordinately bound to the chelators;
a bioactive substance having histidine tags, which are coordinately bound to the metal ions; and
a blocking agent having ligand sets, which are coordinately bound to the metal ions to which the bioactive substance is not coordinately bound.

2. A substrate as defined in Claim 1, wherein:
the histidine tags are peptides having at least six histidine residues which are close to each other.

3. A substrate as defined in either one of Claim 1 and Claim 2, wherein:
the ligand sets of the blocking agent have at least four imidazole groups; and
each molecule of the blocking agent has one of the ligand sets.

4. A substrate as defined in any one of Claim 1, Claim 2, and Claim 3, wherein:
the blocking agent includes one of: water soluble polymer molecules and water soluble proteins;
the water soluble polymer molecules are one of: polyethylene glycol, polymer molecules including phosphorylcholine groups, polysaccharides, polyvinyl alcohol, and polyhydroxy ethyl methacrylate; and
the water soluble proteins are one of: albumin and casein.

5. A substrate as defined in Claim 4, wherein:
the ligand sets of the water soluble proteins are imidazole groups derived from histidine.

6. A substrate as defined in any one of Claims 1 through 5, wherein:
the chelators are three dimensionally covalently bound to the base material via a hydrogel; and
the hydrogel is a carboxylic acid derivatives of dextran.

7. A substrate as defined in any one of Claims 1 through 6, wherein:
the chelators are nitrilotriacetic acid derivatives.

8. A substrate as defined in any one of Claims 1 through 7, wherein:
the surface of a single substrate has both portions to which the bioactive substance is coordinately bound and portions to which the bioactive substance is not coordinately bound.

9. A method for producing a substrate as defined in any one of Claims 1 through 8, comprising the steps of:
causing the bioactive substance to contact the chelators, to which the metal ions are coordinately bound, to coordinately bind the bioactive substance to the metal ions; and
causing the blocking agent to contact the metal ions, to coordinately bind the blocking agent to the metal ions.

10. A method for producing a substrate as defined in Claim 9, wherein:
the concentration of the blocking agent when it is caused to contact the metal ions is within a range from O.lnM to 20µM.


SEQUENCE LISTING
<110> FUJIFILM Corporation
<120> Substrate and method for producing the substrate
<150> JP2008-232840
<151> 2007-09-20
<160> 6

<210> 1
<211> Length 6
<212> PRT
<213> Artificial Sequence
<223> Synthetic peptide
<400> His His His His His His
1 5

<210> 2
<211> Length 8
<212> PRT
<213> Artificial Sequence
<223> Synthetic peptide
<400> His His His His His His His His
1 5

<210> 3
<211> Length 10
<212> PRT
<213> Artificial Sequence
<223> Synthetic peptide
<400> His His His His His His His His His His
1 5 10

<210> 4
<211> Length 20
<212> PRT
<213> Artificial Sequence
<223> Synthetic peptide
<400> His Ser His Ser His Ser His Ser His Ser
1 5 10
His Ser His Ser His Ser His Ser His Ser
15 20

<210> 5
<211> Length 12

<212> PRT
<213> Artificial Sequence
<223> Synthetic peptide
<400> His Lys His Lys His Lys His Lys His Lys His Lys
1 5 10

<210> 6
<211> Length 12
<212> PRT
<213> Artificial Sequence
<223> Synthetic peptide
<400> His Cys His Cys His Cys His Cys His Cys His Cys
1 5 10